# EUROPEAN PATENT APPLICATION

(11) **EP 4 838 533 A1**
(43) Date of publication of application: **11.11.2026**
(21) Application number: 25174273.0
(22) Date of filing: 05.05.2025
(51) Int. Cl.: G16C 20/10, G16C 20/30

(54) **METHOD FOR ASSISTING IN GENERATING A RECIPE FOR A RIGID POLYURETHANE FOAM**

(71) Applicant: BASF SE, 67056 Ludwigshafen am Rhein (DE)
(72) Inventor: ELING, Berend, 49448 Lemfoerde (DE); DAISS, Andreas, 67061 Ludwigshafen am Rhein (DE); PFEIFLE, Mark Claudius Gerhard, 67061 Ludwigshafen am Rhein (DE); DEGLMANN, Peter, 67056 Ludwigshafen am Rhein (DE); SCHMIDT, Sonja, 67117 Limburgerhof (DE); SCHUETTE, Markus, 49448 Lemfoerde (DE)
(74) Representative: BASF IP Association

(57) **Abstract**

The invention refers to a method for assisting in generating a recipe for a rigid polyurethane foam. A start recipe is associated with one or more ingredients, wherein the ingredients comprise ingredients of a polyol-component, and ingredients of an isocyanate-component comprising an isocyanate. Material properties of the polyol and/or the polyol-component are determined based on the start recipe. The start recipe is validated based on the determined material properties of the polyol and/or the polyol-component. If the validation result is positive, process parameters associated with the parameters utilized in a foaming process are received. Foam characteristics are determined based on the validated start recipe and the process parameters. Application properties of the rigid polyurethane foam are determined based on the determined foam characteristics. The application properties are provided for assisting in generating a final recipe for a rigid polyurethane foam comprising target application properties.

## Description

### FIELD OF THE INVENTION

The invention refers to a method, an apparatus and a computer program product for assisting in generating a recipe for a rigid polyurethane foam comprising one or more target application properties.

### BACKGROUND OF THE INVENTION

Rigid polyurethane foams are utilized in many applications since they can be adapted to many requirements. However, since rigid polyurethane foams are so adaptable it can be difficult, time-consuming and resource-intensive to develop a rigid polyurethane foam suitable for a given application.

### SUMMARY OF THE INVENTION

Rigid Polyurethane (PU) foams show a unique property combination of high compression strength and low thermal conductivity. Therefore, they are widely used in insulation applications like refrigeration, lamination, and pipe insulation. The good thermal insulating properties result from a combination of a fine, closed-cell foam structure and cell gases that are poor heat conductors. Rigid PU foams are highly cross-linked, closed-celled materials produced by reacting organic polyisocyanates with one or more compounds having two or more reactive hydrogen atoms in the presence of blowing agents, catalysts and optionally auxiliaries and/or additives. The compounds used in the production of polyurethanes for their two or more isocyanate-reactive hydrogen atoms are preferably polyether alcohol and/or polyester alcohols. These polyols are selected with regard to costs and the desired performance characteristics. The mechanical properties and the processing characteristics of the rigid PU foam can be adjusted over a wide range using several available raw material components and combinations thereof. Foams are obtained by adding physical blowing agents or by the formation of carbon dioxide, which is produced in the reaction of water with the isocyanate groups of the organic polyisocyanate or is produced by formic acid added as blowing agent. Possible organic polyisocyanates are aliphatic, araliphatic and preferably aromatic polyfunctional isocyanates. Preferred polyisocyanates are mixtures of diphenylmethane diisocyanate (MDI) and polyphenylenepolymethylene polyisocyanate (polymeric MDI or PMDI). A typical rigid PU form recipe consists of a polyol- and an isocyanat component. The polyol-component is usually a mixture of different polyols, physical and chemical blowing agents, surfactants and catalysts, as mentioned above. The isocyanate-component comprises usually a mixture mixtures of MDIs and/or PMDI.

Depending on the recipe indicative of the polyol- and isocyanate-component and the processing parameters used for processing the components a resulting foam product can have a plurality of different mechanical and thermal characteristics. Thus, for new applications and products the design process normally comprises an extensive experimental campaign in which different rigid PU foams are produced and tested for one or more application properties in order to find at least one rigid PU foam that fulfils respective targets for the application properties and processability requirements. Besides the composition of the polyol and isocyanate components, sometimes a product development process can also comprise the development of completely new ingredient, e.g. novel polyols with special properties. Thus, the design process is not only time-consuming but also resource-intensive and produces a plurality of waste products. In this context computer-based models have been developed for determining respective application properties of rigid PU foams in advance to reduce the resources and time in developing rigid PU foams for new applications. However, also the utilization of these models is often time-consuming, needs an expert user and requires a high demand on computational resources. Moreover, these models are generally not connected and follow a plurality of different development and design frameworks and application ideas. Thus, utilizing these computational models for determining application properties of rigid PU foams to assist in the design process is often as time-consuming, resource-intensive and driven by expert knowledge as the experimental process.

The inventors have found that first determining material properties for a to be utilized polyol, validating a recipe based on the material properties of the polyol followed by determining foam characteristics and based on the foam characteristics application properties of the polyurethane foam only if the recipe is validated positive, provides a workflow that is effective in assisting in a respective rigid PU foam design process. In particular, since the polyol and the recipe are validated, application properties are not determined for polyol recipes that are not realistic or that do not fulfil the intended properties already at this stage. Thus, the described workflow allows to assist an operator, in particular, a chemist, in finding a suitable polyol and/or rigid PU foam recipe with respective target application properties in a faster, more efficient, in particular, less experimental and computer-resource intensive, way.

In a first aspect a method, in particular, a computer implemented method, is presented for assisting in generating a recipe for a rigid polyurethane foam comprising one or more target application properties, wherein the method comprises a) receiving a digital representation of a start recipe for producing a rigid polyurethane foam, wherein the start recipe is indicative of one or more ingredients utilizable for producing the rigid polyurethane foam, wherein the ingredients comprise ingredients of a polyol-component comprising a polyol and a blowing agent and ingredients of an isocyanate-component comprising an isocyanate, b) determining one or more material properties of the polyol and/or the polyol-component based on the start recipe, c) validating the start recipe based on the determined one or more material properties of the polyol and/or the polyol-component, d) if the validation result is positive, receive one or more process parameters associated with the parameters utilized in a foaming process of the polyurethane foam, e) determine one or more foam characteristics based on the validated start recipe and the one or more process parameters, wherein the one or more foam characteristics are associated with structural and compositional characteristics of the rigid polyurethane foam, f) determine one or more application properties of the rigid polyurethane foam based on the determined one or more foam characteristics, and g) providing the one or more application properties for assisting in generating a final recipe for a rigid polyurethane foam comprising target application properties.

The method refers in particular to a computer-implemented method and can thus be performed by a general or dedicated computer adapted to perform the method, for instance, by executing a respective computer program. The method can be performed in a distributed computing environment like cloud computing, network computing, etc.

The method is configured for assisting in generating a recipe for a rigid PU foam. In particular, the assisting is performed by determining and providing application properties that can then be utilized for validating if a rigid PU foam is suitable for a specific application, for instance, fulfils one or more of the target application properties. However, the assisting can also be provided by determining whether a rigid PU foam fulfils respective target application properties or by directly providing control signals for production processes including a rigid PU foam. In an embodiment the method is configured for generating a recipe of a rigid Pu foam. In this embodiment the method comprises a step of providing the one or more application properties for generating the recipe for producing the rigid polyurethane foam comprising the one or more properties. The one or more target application properties refer to application property targets that should be met by the application properties of the polyurethane foam in order to be usable in a predetermined application. For example, the target application properties can include thresholds, ranges or single values that should be met by application properties of the rigid polyurethane foam. In this context it is noted that, if not indicated otherwise, in this application the referring to a quantity, for instance, an application property, includes referring to one or more respective values of the quantity. Thus, referring to determining a quantity, for instance, an application property, refers to determining a value for the quantity. Application properties are properties that determine whether a rigid PU foam is utilizable in a predetermined application. Application properties can be measurable by one or more known experimental methods. Preferably, the application properties comprise mechanical properties and/or thermal properties of the rigid PU foam. Preferably, the application properties comprise at least one of compression strength, adhesion of the foam to surfaces, tensile strength, surface quality, long-term insulation properties, foam part deformation over time, and thermal conductivity.

In a first step a digital representation of a start recipe for producing a rigid PU foam is received. For example, the receiving can comprise accessing a database on which a respective recipe is already stored. However, the receiving can also comprise receiving the recipe as user input via a respective user interface. The digital representation represents the start recipe in the digital world and thus makes the start recipe accessible for computational processing. For example, the digital representation can refer to an identifier of a known recipe that allows to access the respective information on the recipe. However, the digital representation can also be a table or a matrix format in which the information of the start recipe for producing the rigid polyurethane foam is provided. The start recipe is a recipe with which the process of generating a final recipe that refers to a PU foam that fulfills the target application properties is started. The start recipe is associated with a start PU foam. The start recipe can be arbitrary or can be chosen from a list of recipes that are associated with PU foams that have a known potential of having respective application properties. During the method for generating the final recipe, the start recipe can be modified, if necessary, leading to a modification of the associated PU foam and thus to different application properties. However, the start recipe can also after the method turn out as the final recipe without modification. The start recipe is indicative of one or more ingredients utilized for producing a rigid PU foam, which is regarded as a start PU foam, and optionally also of the amounts utilized for producing the rigid PU foam, wherein the amounts may be provided as relative amounts, for instance, as relations provided with respect to a predetermined amount, or as absolute amounts. The ingredients indicated by the recipe comprise at least one of a polyol-component comprising a polyol and a blowing agent, and an isocyanate component comprising an isocyanate. Optionally, further ingredients like catalysts, additives, surfactants, etc. can also be indicated by the recipe. Preferably, the recipe comprises the components constituting the polyol-component and the isocyanate-component. If applicable, the recipe can further comprise the relative or absolute amounts of components of a polyol-component and/or an isocyanate-component. A polyol is an organic compound containing multiple hydroxyl groups and can be synthesized from a set of starting materials.

In the next step one or more material properties of the polyol and/or the polyol component are determined based on the start recipe. Determining material properties of the polyol and/or the polyol-component can comprise determining the material properties of the single polyol, a mixture of polyols included in the polyol-component or the entire polyol-component of the recipe. The material properties can be any properties of the polyol and/or polyol-component that allow to validate whether the polyol and/or polyol-component is suitable to be utilized in a recipe for producing a rigid PU foam. Preferably, the material properties are determined for the polyol-component. Preferably, the polyol and/or polyol component properties comprise at least one of a blowing agent solubility for one or more predetermined blowing agents, a viscosity, an isocyanate compatibility with one or more predetermined isocyanates, and a glass transition temperature of the unfoamed polymer. The glass transition temperature is preferably determined for an unfoamed bulk-polymer with a predefined standard recipe. A isocyanate compatibility can be defined as the miscibility of the polyol and/or polyol-component with the isocyanate. In particular, the material properties can be determined with respect to the other ingredients indicated by the start recipe. For example, a blowing agent solubility can be determined with respect to the one or more blowing agents indicated by the start recipe. Also an isocyanate compatibility can be determined with respect to the isocyanate indicated by the start recipe. However, since in many cases the blowing agent and isocyanate are the same for many applications also the commonly utilized isocyanates and blowing agents can be predetermined by default and the respective material properties can be determined with respect to these predetermined ingredients. The determining of the one or more material properties of the polyol and/or polyol-component based on the start recipe can comprise accessing a database and/or a library on which a plurality of material properties for known polyols and/or polyol component are already stored and providing the already stored one or more material properties. However, for mixtures of polyols or new polyols utilized in the polyol-component the determining can comprise utilizing a polyol property model. If at least some polyols of a new mixture are known, the known properties for these polyol can be retrieved from a database and/or library and the properties of the remaining polyols can be determine utilizing the polyol property model. In this case the material properties can be determined by the polyol property model by combining the material properties of the known and unknown polyols in the polyol components, for instance, using respective known relations. A polyol property model can utilize one or more input parameters associated with the polyol and/or polyol-component to determine the one or more material properties. For example, the polyol property model can be based on known physical and/or chemical, empirical, mechanistic, and/or statistical relations and laws that can also be found empirically to derive the respective one or more material properties from parameters associated with the polyol, optionally, taking one or more further ingredients indicated by the and/or polyol-component into account. However, the polyol property model can also be at least partly based on machine learning algorithms that comprise one or more trainable model parameters that are determined based on a historical data set comprising parameters associated with the polyol for at least one polyol and respective one or more material properties of the polyol. Preferably, a machine learning based physical model is utilized that utilizes a known physical, empirical, mechanistic, and/or statistical relation between one or more of the material properties and parameters associated with the polyol that comprises one or more trainable model parameters that are fitted using a historical training data set. The utilized relation can also be a learned relation between quantities or can be a relation found empirically. The trainable parameters can for instance, represent proportional constants or other parameters of respective relations. Preferably, the training can include fitting the trainable parameters based on the training data set. Preferably, the input parameters are chemical composition parameters that are defined by the chemical composition of the polyol. In an embodiment, the determining of the material properties comprises determining a) a chemical composition of the polyol in the polyol component based on the start recipe and b) determining the one or more material properties based on the chemical composition. Preferably, the input parameters for a respective polyol property model are determined based on the determined chemical composition of the polyol. Preferably, determining the chemical composition comprises determining the types of monomers of the polyol and the amount of the respective types of monomers in the polyol. The input parameters can then be the amount of respective types of monomers in the polyol and the polyol property models can be configured to determine the one or more material properties, preferably a blowing agent solubility, a viscosity, an isocyanate compatibility and a glass transition temperature of the resulting polyurethane formed from that polyol based on the amount of the types of monomers in the polyol.

In a next step the start recipe is validated based on the determined material properties of the polyol and/or polyol-component. The validating can comprise validating the start recipe with respect to one or more predetermined criteria based on the determined material properties. The validating can comprise comparing the material properties with one or more target material properties. For example, a target property can refer to a threshold for a solubility of the foaming agent in the polyol. For example, a criterion can be a compatibility between the polyol and the isocyanate. The validating of the start recipe can comprise comparing a determined viscosity of the polyol with a predetermined target viscosity range for the polyol. A validation result is generally positive if the one or more predetermined criteria are fulfilled, for instance, if the one or more predetermined target ranges of a material property are met by the determined property.

In a further embodiment the one or more determined material properties comprise a shelf life determined for the polyol-component and the validating further comprises taking the shelf life of the polyol-component into account. For example, the validating can comprise comparing the determined shelf life with a shelf life threshold that has to be exceeded by the polyol-component to be usable in a respective application. The shelf life can be determined for the polyol-component by taking the further ingredients into account when determining the shelf life of the polyol-component. This is in particular relevant if a polyol-component comprises a hydrofluoroolefins (HFO) type blowing agent. Amine catalysts for PU foam synthesis can degrade HFO blowing agents via nucleophilic attack. The catalysts are consumed by this unwanted reaction, and the lower catalyst concentrations lead to longer gelling times and undesired foam properties. The reactivity towards HFOs can depend on the chemical structure of the catalyst. This reaction reduces the shelf life of the corresponding polyol-component in which the blowing agent and catalysts are already mixed with the polyol and potentially stored for extended periods of time before usage. Thus, preferably, when determining the material properties of the polyol-component a HFO degradation potential for various amine catalysts, e.g. with Solstice LBA as blowing agent, is taken into account. The material property can then refer to how many days the polyol-component can be stored before the amount of catalysts is reduced below a predetermined threshold.

If the validation result is positive, for instance, if the material properties of the polyol and/or polyol-component fulfil predetermined criteria, the start recipe can be regarded as validated start recipe and one or more process parameters are received. For example, the process parameter can be received by accessing a respective database on which the process parameters are stored. However, the process parameters can also be received via a user interface, for instance, by prompting a user via the user interface to input the respective process parameters. The process parameters are indicative of the parameters utilized in a foaming process of the PU foam. The process parameters can refer to any quantities of a foaming process of a PU foam that are adaptable. Preferably, the one or more process parameters comprise at least one of a pressure, a temperature, a pressure profile, an injection pressure, an injection temperature, a mold temperature and a temperature profile utilized during the foaming process. The term "validated start recipe" indicates that the start recipe has fulfilled the criteria of the polyol validation step, but is still the same as the start recipe. Thus, the term is only utilized to indicate the progress the recipe has made in the method steps but not for indicating a change in the start recipe itself.

The method then comprises determine foam characteristics based on the validated start recipe and the one or more process parameters. Foam characteristics are indicative of structure and compositional characteristics of the polyurethane foam. The foam characteristics can be determined for after the end of the foaming process but also for one or more predetermined time points during the foaming process. The foam characteristics can comprise parameters indicative of a microstructure of a resulting polymer and its mechanical properties. Preferably, the foam characteristics comprise at least one of a foam density, a degree of cross-linking, a cell gas composition, cell gas pressure, an average foam cell size and cell shape. The foam characteristics can be determined based on known physical and/or chemical laws, empirical, statistical and/or mechanistic relations. For example, the density of the foam can be determined based on an absolute amount of polyol and isocyanate injected in a respective mold indicated by the validated start recipe and the process parameters. An average cell size of the foam cells can be determined based on process parameters indicative of a mixing process of the ingredients of the validated start recipe and by an amount and type of surfactants indicated by the validated start recipe. A shape of the foam cells can be determined by the amount and type of catalysts indicated by the validated start recipe which determine how fast the foam rises in comparison to how fast the foam gels and becomes a shape-stable solid. However, the foam characteristics can also be determined based on a foaming model that can be solely machine learning based or can be a mixture of utilizing physical and chemical laws, empirical, statistical and/or mechanistic relations together with one or more machine learning based parameters. Preferably, the foaming model is a machine learning physical model that is based on a known relation and one or more trainable parameters. The known relation can also be a learned relation between quantities, can be found empirically or statistically based on respective experiments, or derived from physical or chemical laws. The machine learning based parameters can be trained based on a historical training data set comprising process parameters and recipes for at least one polyurethane foam and respectively known foam characteristics. In a preferred embodiment at this stage also one or more foaming characteristics can be determined, for instance, utilizing the foaming model that models the foaming process. Preferably, the foaming characteristics comprise at least one of a foaming time, a gelling time, curing speed, a foaming speed, and a demoulding time. The foaming characteristics can be determined based on the validated start recipe and the one or more process parameters, for instance, in the same foaming model for determining the foam characteristics. Foaming characteristics can provide additional input, and information that can be utilized to validate the suitability of a rigid PU foam for a specific application. For example, long demoulding times might not be desirable for products that are to be produced in high quantities.

Based on the determined one or more foam characteristics of the rigid PU foam one or more application properties can then be determined. Also, in this case the one or more application properties can be determined based on the determined foam characteristics utilizing known physical and/or chemical laws. However, the application properties can be determined based on the foam characteristics utilizing an application property model configured to provide one or more application properties as output when provided with the determined foam characteristics as input. For example, the application property model can be a solely machine learning based model or a machine learning based model that is based on physical and/or chemical laws and relations. Preferably, the property model is a machine learning based physical model based on one or more physical relations. The physical relations can also be a learned relation between quantities or can be found empirically based on respective experiments. The training of the trainable parameters is based on a historical training data set comprising one or more foam characteristics of at least one rigid PU foam and known respective application properties.

The method then further comprises providing the one or more application properties for assisting in generating a final recipe for a rigid polyurethane foam comprising target application properties. For example, the one or more application properties can be configured such that they can directly be implemented into a controlling unit of a design process that validates the one or more application properties and determines whether the provided start recipe of the rigid PU foam fulfils the target application properties and thus is the final recipe. Preferably, the application characteristics are provided for controlling and/or monitoring an application including the polyurethane foam. The application can be a production of the polyurethane foam. The application can be any other application that utilizes the polyurethane foam. In an embodiment, the controlling and/or monitoring of the application can refer to controlling and/or monitoring a demoulding of the polyurethane foam, in particular, the demoulding time. In an embodiment the controlling and/or monitoring of the application can refer to controlling and/or monitoring the application to utilize the determined final recipe for producing the polyurethane foam. Preferably, the providing of the application properties comprises providing control data for controlling and/or monitoring a polyurethane foaming process based on the final recipe and the one or more of the application properties. The control data can be configured to be implemented into a controlling and/or monitoring system of the respective application. Generally, control data refer to any kind of data that allows to derive control and/or monitoring actions for controlling and/or monitoring an application, in particular, a PU foaming process.

In an embodiment, the method comprises, if the validation of the polyol and/or the polyol-component is negative, generating a new start recipe comprising a different polyol-component and performing the steps of the method based on the new start recipe as start recipe. The performed steps can in particular refer to the determining of the one or more material properties of the polyol and/or the polyol-component, the validating of the start recipe based on the determined material properties, and if the validation is positive the steps leading to the determination of a technical application property. In an embodiment, if the validation of the polyol and/or the polyol-component is negative, also other parameters of the start recipe can be changed, for instance, the blowing agent and/or the isocyanate. However, in some applications in which changes of the isocyanate or the blowing agents are not technically or economically sensible, it is preferred that the polyol is changed. Preferably, the method further comprises receiving recipe constraints indicative of constraints with respect to the polyol and/or the polyol-component, wherein the new start recipe is generated based on the received constraints. For example, the constraints can refer to a list of polyols or polyol types that can be utilized as polyols and/or a list of polyols and/or polyol types that should not be utilized. Further, the constraints can refer to constraints of a recipe for the polyol, for instance, constraints with respect to the utilized starting materials, one or more types utilized, etc. When determining the new start recipe the respective constraints can then be taken into account, for instance, a new polyol can be selected from the list of acceptable polyols.

In an embodiment the method further comprises determining one or more insulation properties of the polyurethane foam based on the validated start recipe and the one or more foam characteristics. Preferably, the insulating properties are determine utilizing a type and amount of blowing agents indicated by the validated start recipe. The foam characteristics can refer to at least one of a density of the foam, an average cell size of the foam cells, and a shape of the foam cells. The density of the foam can be determine based on an absolute amount of polyol and isocyanate injected in a respective mold indicated by the validated start recipe and the process parameters. An average cell size of the foam cells can be determined based on process parameters indicative of a mixing process of the ingredients of the validated start recipe and by an amount and type of surfactants indicated by the validated start recipe. A shape of the foam cells can be determined by the amount and type of catalysts indicated by the validated start recipe which determine how fast the foam rises in comparison to how fast the foam gels and becomes a shape-stable solid.

In an embodiment the method comprises receiving one or more target constraints with respect to the one or more application properties, wherein the one or more target constraints are to be fulfilled by the polyurethane foam, wherein the method further comprises validating the validated start recipe with respect to the target constraints based on the determined one or more application properties and providing a final recipe based on the validation preferably, if the validation is negative, a new start recipe is generated comprising at least one of a different polyol-component, catalyst, amount of one or more components and/or process parameter and the steps of the method are performed based on the new start recipe as start recipe, or if the validation is positive, the validated start recipe is provided as final recipe for controlling and/or monitoring a production of the polyurethane foam. It is noted that here the term "validated start recipe" again only indicates that the start recipe has already been subjected to the step of validating the start recipe based on the material properties of the polyol and/or the polyol-component and thus is a realizable recipe. The term "validated" therefore does not refer to the further performed validation process with respect to the target constraints. The one or more target constraints preferably comprise one or more target application properties. However, the one or more target constraints can also comprise further additions, for instance, relations between target application properties. Moreover, the one or more target constraints can also comprise an optimization goal for one or more application properties that should be fulfilled by the polyurethane foam. For example, an optimization goal can comprise minimizing or maximizing a first target property, optionally, with respect to a second target property that should be kept in a respective target range. In such a case the validation can comprise determining whether the optimization goal has been reached, for instance, if the first target property has reached its maximum or minimum within the target range of the second target property. Moreover, for multi target optimization, in which a plurality of application properties are to be optimized with respect to each other, the validation can also comprise determining whether the recipe is, for instance, Pareto optimal with respect to the to be optimized application properties.

Preferably, the provided final recipe is indicative of, in particular, comprises, control data for controlling and/monitoring the polyurethane production process.

In a further aspect an apparatus is presented for assisting in generating a recipe for a rigid polyurethane foam comprising one or more target application properties, wherein the apparatus comprises one or more processors configured to perform a method comprising a) receiving a digital representation of a start recipe for producing a rigid polyurethane foam, wherein the start recipe is associated with one or more ingredients utilizable for producing the polyurethane foam, wherein the ingredients comprise ingredients of a polyol-component comprising at least a polyol, and a blowing agent, and ingredients of an isocyanate-component comprising an isocyanate, b) determining one or more material properties of the polyol and/or the polyol-component based on the start recipe, c) validating the start recipe with respect to one or more predetermined criteria based on the determined material properties of the polyol and/or the Ppolyol-component, d) if the validation result is positive, receive one or more process parameters associated with the parameters utilized in a foaming process of the polyurethane foam, e) determining foam characteristics based on the validated start recipe and the one or more process parameters, wherein the foam characteristics are associated with structural and compositional characteristics of the polyurethane foam, f) determining one or more application properties of the polyurethane foam based on the determined foam characteristics, and g) providing the one or more application properties for assisting in generating a final recipe for a polyurethane foam comprising target application properties.

In a further aspect a computer program product is presented configured for causing an apparatus as described above to perform a method as described above when the computer program product is carried out by the apparatus.

It shall be understood that the methods as described above, the apparatuses as described above and the computer program products as described above have similar and/or identical preferred embodiments, in particular, as defined in the dependent claims.

It shall be understood that a preferred embodiment of the present invention can also be any combination of the dependent claims or above embodiments with the respective independent claim.

These and other aspects of the present invention will be apparent from and elucidated with reference to the embodiments described hereinafter.

### SHORT DESCRIPTION OF DRAWINGS

- Fig. 1 to 4: show schematically and exemplarily different embodiments of the workflow of the method for assisting in generating a recipe for a rigid polyurethane foam,
- Fig. 5: shows schematically and exemplarily an overview over a network of models that can be utilized in the workflow, and
- Fig. 6: shows schematically and exemplarily a polymer network made up of polyol molecules and isocyanate molecules of different functionalities.

### DETAILLED DESCRIPTION OF EMBODIMENTS

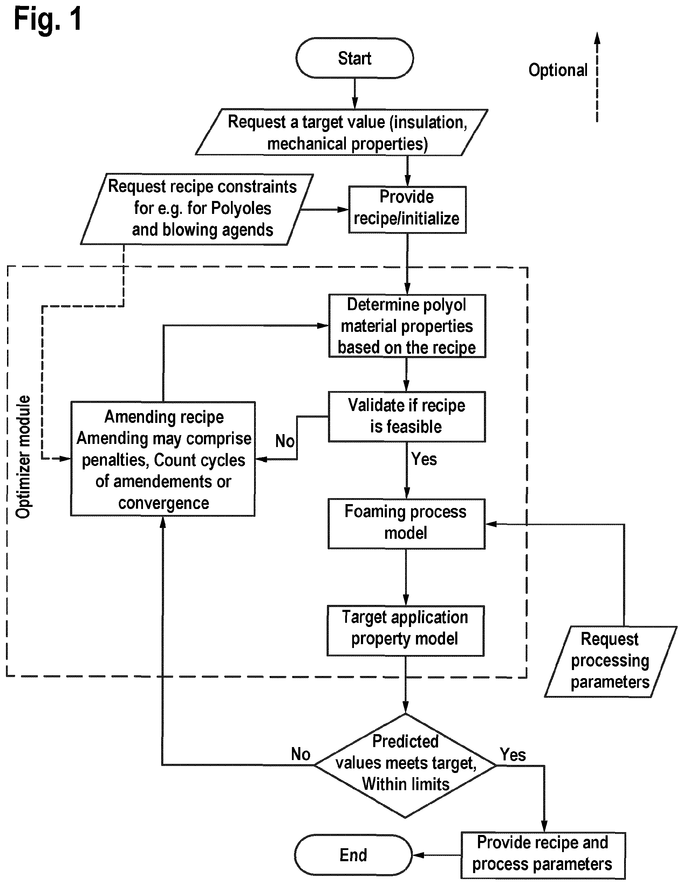

Fig. 1 shows schematically and exemplarily an embodiment of a workflow representing a method for assisting in generating a recipe for a rigid PU foam. In this exemplary workflow first a target value as target constraint for at least one target application property is requested, for example, for an insulation property or a mechanical property of the rigid PU foam. For example, the requesting can be realized by providing a respective user interface to a user workstation into which a user can then input the respective target values. For example, the respective target application properties can be selected by a drop down menu and the values for the respective selected target application properties can then be provided via a user input device. However, instead of a target value as in the following example, also an optimization goal for one or more application properties can be defined as target constraints. Further, recipe constraints can be requested, for example, for the polyols and blowing agents utilized in a recipe. Also in this case the requesting can comprise causing a user interface to request the respective constraints from a user or interact with a user in another way. However, the request can also comprise accessing a database on which respective determined or default constraints are already provided. The recipe constraints can determine, for instance, which polyols, blowing agents and/or isocyanates can be utilized in the recipe. Further, the recipe constraints can also refer to the utilization of specific catalysts, additives or surfactants and also can determine the maximum or minimum amounts of ingredients to be utilized. Further, the recipe constraints can refer to constraints in the to be utilized process parameters like a foaming process temperature or pressure. For example, temperature ranges and pressure ranges can be defined as constraints.

Based on the provided recipe constraints a respective start recipe can be provided, for instance, also by requesting a respective start recipe from the user or by automatically generating a start recipe from the constraints and respective predetermined rules. Based on the respective start recipe the method can then be initialized. In the next step material properties of the polyol and/or the polyol-component can be determined based on the start recipe. For example, a database can be accessed on which experimental determined material properties of a plurality of polyols and/or the polyol-components are already stored and the material properties can be determined by selecting the material properties of the respective polyol. However, for unknown polyols or for polyol mixtures also material models can be utilized that can be based on physical and/or chemical laws and utilize one or more characteristics of the polyol or polyols as input to determine a respective material property. For example, the type and amount of monomers of a polyol can be utilized to predict a viscosity, solubility of the blowing agent and/or compatibility with an isocyanate. Alternatively to models that are purely based on physical and/or chemical laws also purely machine learning based models or mixed models that comprise machine learning and physical law based parts can be utilized. Respective training data sets can comprise polyol characteristics of one or more polyols and corresponding material properties of the polyol. More details with respect to possible models and methods for determining the polyol material properties will be given further below. In case of polyol-components comprising more than one polyol, already known properties can be determined by accessing a database, while for unknown polyols the respective polyol material model can be utilized to determine a respective material property. Based on the material properties of the respective polyols included in the polyol-component the polyol model can then be configured to utilized known physical and/or chemical laws to generate the material model of the polyol-component.

In a next step it is validated if the start recipe is technically feasible based on the material properties of the polyol. For example, it is determined if the polyol is compatible with the isocyanate, whether the polyol fulfils viscosity requirements for the rigid PU foam processing and whether the solubility of the blowing agent in the polyol is within an acceptable range. The validation can comprise, for instance, comparing the respective material properties to known or predetermined constraints for these material properties. If the result of the validation indicates that the start recipe is not feasible the start recipe can be amended. In particular, the polyol or its amount can be changed. However, also the blowing agent or in some cases also the isocyanate can be changed. When amending the start recipe respective recipe constraints can be taken into account. The amending can comprise determining a penalty score, count the cycles of amendments and/or determine a respective convergence of the method in order to provide an abort criterion for cases in which no possible recipe can be found with the provided constraints. Based on the amended start recipe that refers to a new start recipe again the polyol material can be determined.

If the validation of the start recipe is positive, e.g. if the start recipe is feasible, in a next step a foaming process model can be utilized to model the foaming process and to determine foam characteristics for the rigid PU foam that result from the foaming process. Since the foam characteristics depend on the foaming process, respective process parameters can be requested in this step, for instance, again by prompting a user in a user interface to input the respective processing parameters. However, also standard or default processing parameters can also be stored on a database and can be utilized. The processing parameters can for instance be indicative of temperature profiles, for instance, of the injection and/or the mold, and/or pressure profiles utilized during the foaming process. Moreover, the processing parameters can also be already part of the start recipe. In this embodiment the receiving can comprise deriving the processing parameters from the start recipe. Further, the foaming process model can utilize the start recipe, in particular the ingredients and amounts of ingredients, for example the amount and type of catalysts, associated with the start recipe to model the respective foaming process and determine the foam characteristics. The foam characteristics can refer, for instance, to a foam density, a degree of cross-linking of the foam, a cell gas composition, an average foam cell diameter, a cell shape, or a cell gas pressure. Also the foaming process model can be based on physical and/or chemical laws or can be purely machine learning based or can be a mixture in which some parameters are trainable but also physical and/or chemical relations are utilized. More details on the model will be provided further below.

Based on the determined foam characteristics a target application property model can be utilized to determine the one or more target applications, in particular, mechanical and/or thermal properties of the rigid PU foam. Preferably, the target application property model can determine, based on the foam density, the degree of cross-linking, the cell gas composition and the cell gas pressure, an average foam cell diameter, a cell shape, insulating properties and/or a compression strength. In particular, the target application property model can be a model that models both mechanical and thermal properties. Details on the respectively utilizable models will be given further below. The respectively determined target application property can then be provided to a user, for instance, via the user interface in order to assist in the determining of a suitable recipe for a respective target application.

In a preferred embodiment as shown in Fig. 1 the method can further comprise automatically determining if the values of the target application properties meet the respective target values within respective limits, as a validation step. If the respective targets are not met the method can again go to amending the start recipe as already described above and again starting the process of determining the application properties with a new start recipe. This leads to a respective iteration until a final recipe is found that meets the target values or another abort criterion is met, for instance, the amount of cycles exceed the predetermined threshold and the method is aborted without a final recipe as result. If a start recipe meets the respective target values the start recipe as final recipe can be provided together with the process parameters for being produced. If more than one recipe, for instance, a list of start recipes fulfils the respective target all start recipes can be provided as final recipes in form of a ranked list that has in accordance with how much the respective final recipes fulfil the targets. For example, based on the comparison between the determined values and the target values a respective validation score can be determined and utilized for ranking different final recipes in accordance with the criteria of fulfilling the target values. The provided final recipes can be provided such that they can be implemented in a controlling and/or monitoring process for producing the rigid polyurethane foam resulting from the recipe. Moreover, also control data can be generated based on the provided final recipe that directly allow to control and/or monitor a respective production process of the rigid PU foam.

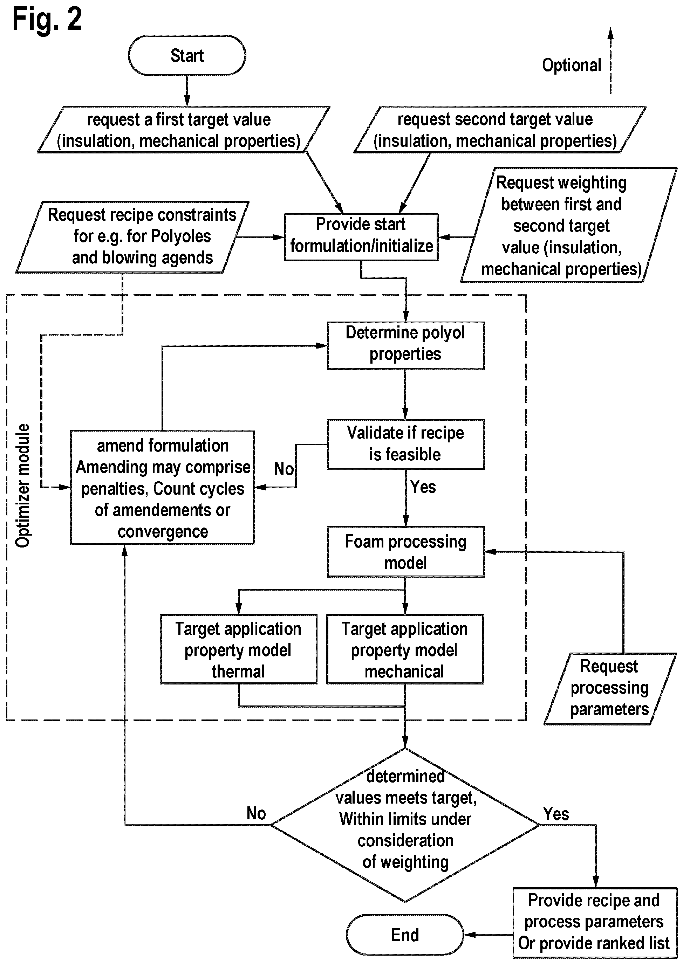

Fig. 2 shows a further embodiment schematically and exemplarily represented as a workflow of the method for assisting and determining a recipe for a rigid PU foam. Since most steps are similar to the embodiment described with respect to Fig. 1, only respective differences of the workflow shown in Fig. 2 to the workflow shown in Fig. 1 will be discussed in the following. In this embodiment, a first and a second target value as target constraints are requested together with a preferred weighting between the first and second target value. For example, for a predetermined application a certain insulation value might be more important than a certain compression strength wherein this importance can be reflected by respectively provided weights. Thus, the weighting can be provided to reflect the respective importance of the target values, respectively. Generally also in this case instead of target values respective optimization goal could be provided for one or more of the target application properties. In this case the weights can determine a prioritization during optimizing the respective application properties. For example, it can be first optimized for the application property with the highest weight and then respectively for application properties with lower weights. The weights can also be utilized to guide a Pareto optimization, for example, to find a solution on the Pareto front that takes the respective weighting of the plurality of optimization goals into account. The method is then continued in the same way as described with respect to Fig. 1. The requested weights are then utilized in the last validation step to determine whether or not the start recipe fulfils the predetermined targets. In this embodiment, the target application property model can be split into two parts where each of the model parts determines a different type of application property, for instance, one part determines the thermal application properties and the other determines the mechanical application properties as shown the example in Fig. 2. For validating the start recipe, the first and second target value has to be met wherein the respective weights are considered when determining if the rigid PU foam meets the predetermined target values. If the respective targets are not met the method can go to amending the start recipe as already described above and again to starting the process of determining the application properties with a new start recipe. This leads to a respective iteration until a recipe is found that meets the first and second target values or another abort criterion is met, for instance, the amount of cycles exceed the predetermined threshold, and the method is aborted without a final recipe as result. If a recipe meets the respective target values the recipe can be provided as final recipe together with the process parameters for being produced.

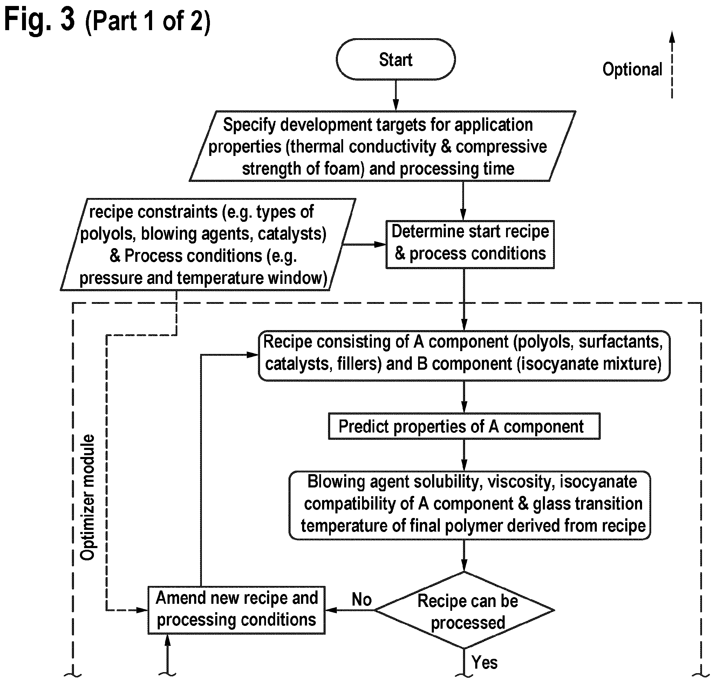

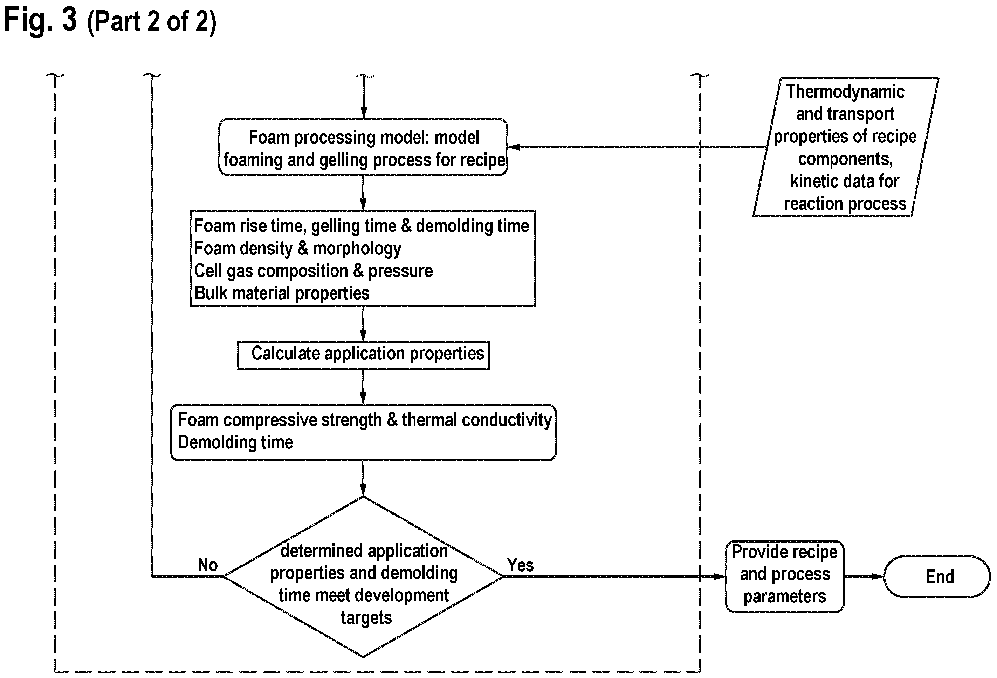

Fig. 3 shows schematically and exemplarily a further embodiment of a workflow of the method for determining a recipe for a rigid PU foam, wherein this embodiment shows some preferred details. Also in this case the description will be focused on the differences between the embodiment shown in Fig. 3 and the embodiment shown already in Figs. 1 and 2. In these exemplary embodiments the target application properties refer to development targets for thermal conductivity and compressive strength of the rigid PU foam and further to a processing time, for instance, to a demolding time. The demolding time can be of interest for the production of the rigid PU foam. For example, products that are produced in large quantities are generally preferred having a short demolding time whereas for products that are produced, for instance, in low quantities or as single pieces, longer demolding times could be acceptable. In this embodiment, the requested recipe constraints can exemplarily refer to the types and amounts of polyols, blowing agent and catalysts and can also refer to the process conditions, for instance, the process parameters like a pressure profile or a temperature profile. Based on this in addition to the start recipe respective process parameters can be determined and thus do not have to be requested as described above. In this embodiment the ingredients provided by the start recipe are separated into an polyol-component or component mixture that refers to the polyols, surfactants, catalysts and fillers provided by the start recipe and a isocyanate-component or component mixture that refers to the isocyanate or an isocyanate mixture. Since in this embodiment the start recipe is indicative of more than the polyol also in addition to the material properties of the polyol the material properties can be determined for the other constituents of the polyol-component, meaning that the material properties can take into account the influence of the surfactants, catalysts and fillers on the polyol. In this preferred embodiment the determined material properties refer to a blowing agent solubility, a viscosity and an isocyanate compatibility of the polyol-component and further to a glass transition temperature of the final polymer derived from the polyol. Also in this case the next step refers to a validation of the start recipe based on the determined material properties. If the recipe is validated negative in this case in addition to the amendment of the ingredients of the recipe also the processing conditions can be amended. In addition to the above embodiments of Figs. 1 and 2 thermodynamic and transport properties of recipe components and connective data for a reaction process are further taken into account in a foaming process model that models the foaming and gelling process for the respective recipe. The thermodynamic and transport properties can either be determined based on already existing databases for the recipe components and ingredients or can also be determined utilizing respective models. The foam and/or foaming characteristics determined in this case from the foaming process model refer to a foam rise time, a gelling time, a demolding time, a foam density, a foam morphology, a cell gas composition and a cell gas pressure. Further, also bulk material properties can be determined. Based on these determined foam characteristics in this case the application properties determined are the foam compressive strength and thermal conductivity. Further, the demolding time determined as foam characteristic is also utilized for validating whether the recipe fulfils the respective target values provided above.

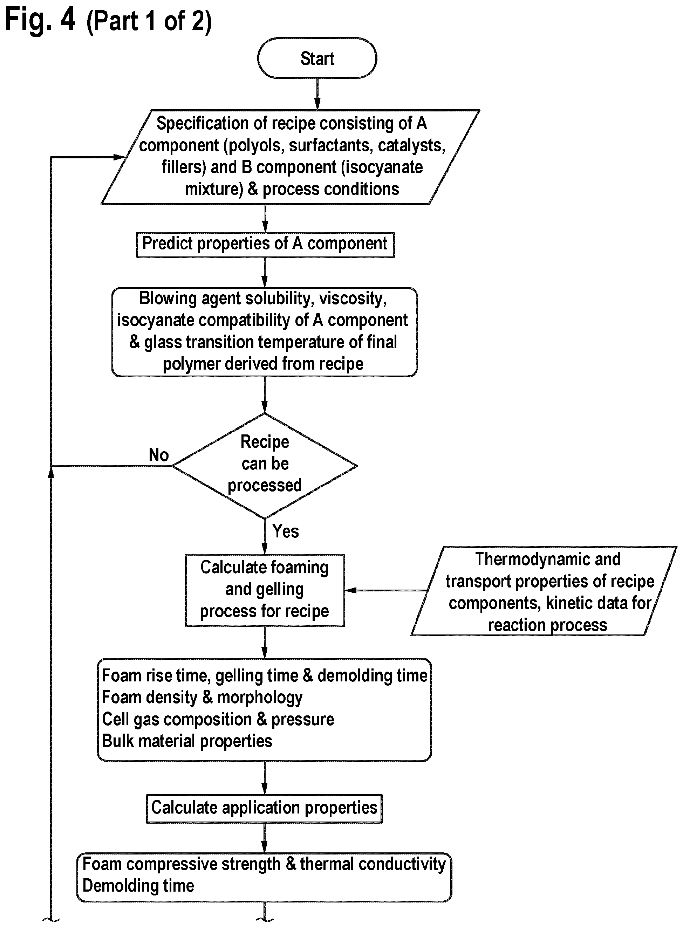

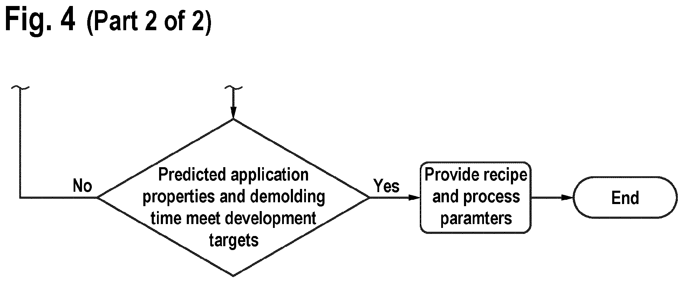

Fig. 4 shows schematically and exemplarily a further embodiment of a workflow of the method for determining a recipe of a rigid PU foam that is similar to the embodiment shown in Fig. 3. In particular, the main difference between the embodiments shown in Fig. 3 and in Fig. 4 refers to not automatizing the iteration and thus a providing of new start recipes and process parameters if performed in a human interaction. In the embodiment shown in Fig. 4, if a validation is negative in one of the validation steps, for instance, a user can be notified and the user can then provide a respective new start recipe and/or new process parameters. Thus, in these embodiments the requesting of constraints and the automated determination of amended recipes can be omitted.

In the following some possible realizations of the respective models utilized in the above workflows will be provided in more detail.

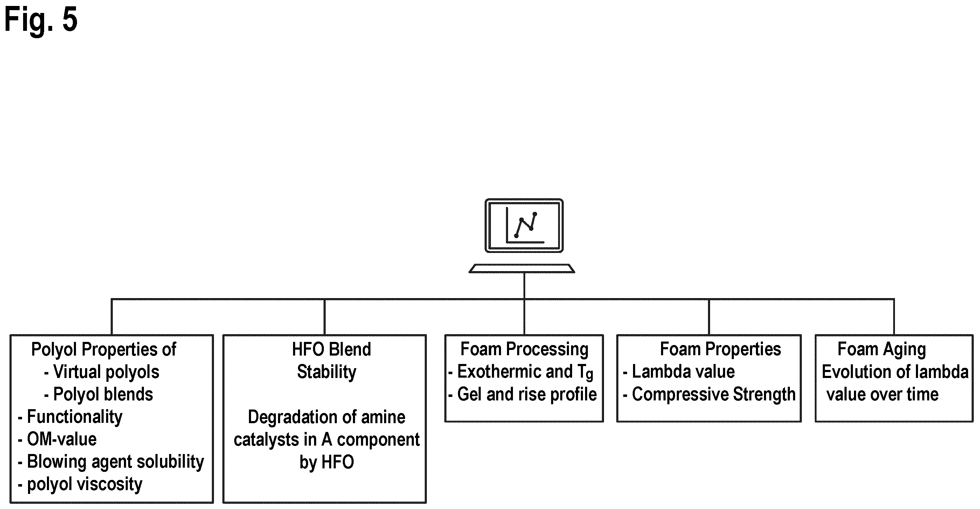

In the following some more detailed preferred embodiments of respective models that can be utilized in the above method are described. The method can utilize different models for determining different aspects of the rigid PU foam as described above. Preferably, a network of models as shown in Fig. 5 can be utilized. The models can comprise a polyol property model configured for determining polyol properties of polyols and polyol blends from existing polyols and optionally from characteristics of new polyols. The following parameters can be determined: blowing agent solubility, viscosity, isocyanate compatibility and/or the glass transition temperature of a model foam, for instance, with polymeric MDI at an Index of 100. A stability model can be utilized for determining the stability and thus shelf life of the polyol-component of the PU foam including a polyol and at least one of a blowing agent and a catalyst and optionally, surfactans, etc. Preferably, the polyol-component comprises a blowing agent, preferably, a HFO blowing agent like Solstice LBA. Further the models can comprise a foaming process model that can determine a rise and gel time, a demolding time, a demolding cell gas pressure, time dependent profiles of temperature, end group conversion, crosslink density, and foam density. Further an application property model can be utilized that is configured to determine application properties of the rigid PU foam, for example, the compression strength and thermal conductivity as a function of the density, wherein optionally also cell anisotropy can be taken into account. Further the models can comprise an aging model that is configured to determine an aging of the PU foam.

In the following a possible polyol property model for determining a viscosity of the polyol-component is described. If historical training data is utilized for training one or more parameters of the model the training data can be extracted from the literature. A possible relation that can be utilized as basis for a model for determining the viscosity *η* of a polyether polyol can be: $η = \frac{f}{OHV} exp \left(C+\frac{k}{T - {T}_{0}}+\frac{E}{T}\right)$

In this equation, *f* represents the polyol functionality, e.g. number of OH and NH groups per molecule, whereas C, *k* and E are trainable parameters. OHV is the hydroxyl value of the polyol, i.e. the mass of potassium hydroxide (KOH) required to neutralize the acetic acid which is consumed upon acetylation of one gram of the polyol (usually given in mg KOH per gram of sample). Thus, this quantity represents the concentration of hydroxyl groups in the sample, and is inversely correlated with the molecular weight of the polyol (for constant functionality *f*). More data with respect to the equation can be found in the article "The Temperature Dependence of Relaxation Mechanisms in Amorphous Polymers and Other Glass-forming Liquids" by M. L. Williams, R. F. Landel, J. D. Ferry, J. Am. Chem. Soc. 1955, 77, 3701. Also, the temperature T₀ can be fitted based on training data from experimental viscosities. In this equation, three starter dependent trainable parameters enter into the equation. A starter refers to a starting material from which the polyol is generated via propoxylation of a OH- or NH-functional starter. For example, the molecular weight of a polyol is determine by the OHV and the functionality of the starter. Thus, for training a model based on this relation requires a respective amount of training data and thus experimental data.

To reduce the amount of training data required alternatively the following model can be utilized that depends more strongly on established physical relations for macromolecules, in order to be able to determine also the viscosity of polyols for which no training data is available so far. The preferred model can be based on the behavior of viscosities for polymers and their concentrated solutions: $η = F \left({M}_{W}, T\right) ⋅ ξ \left(ρ, T, chemistry\right)$

Here, *F*(*M_{W}, T*) is a "structure sensitive factor", which depends on molecular weight and coiling of chains, and *ξ*(*ρ, T*) is called the "friction factor" that covers the oligomer-specific chemistry, e.g. directed interactions like hydrogen bonding, as well as the temperature dependence and density ρ. In this relation, if only one class of oligomers well below critical conditions is considered only a chemistry dependent *ξ*(*T*) can be trained based on a training data set. Thus, a much smaller training data set can be utilized for the training.

If linear oligomers well below a critical molecular weight required for the occurrence of entanglements are considered, it can be expected that *F*(*M_{W}, T)* depends linearly on molecular weight (*MW*)*.* As the considered polyols of differing functionality represent star like structures, it should however rather be assumed that the proportionality is to the molecular weight of single branches, and - if the central part of the star like structure can be neglected - this means that the following formula can be utilized for such cases: $log \left({η}_{structure}\right) ∝ log \left(c\frac{{M}_{W}}{f}\right) \times exp \left(-d\times \left(T-{T}_{offset}\right)\right)$ $log \left({η}_{structure}\right) = log \left({M}_{W}\right) - log f - d \times \left(T-{T}_{offset}\right) + const$

For many polyols with higher functionality, however, it is observed that *η* decreases over a large range of the lower *M_{W}* or even over the entire range of *M_{W}* considered. The temperature dependence of log(*η_{structure}*) is taken into account by the two constants d and *T*_{offset}.

An inspection of the available data suggests that an assumption of a monotonous increase of *η_{c}* with OHV leads to an excellent agreement for the dependence of viscosity vs. molecular weight for a given starter and temperature. Thus, the following overall expression can be utilized: $log \left(η\right) = log \left({M}_{W}\right) - log f + a ⋅ {OHV}^{n} + b$

Parameters n, a and b can be obtained by plotting log(*η* ) - log(*M_{W}*) + log *f* vs. *OHVⁿ.* At least for temperatures around 25°C, this simple formula is able to cover viscosity dependence on molecular weight for each starter quite accurately.

A temperature dependency can be taken into account by determining a temperature dependence of a and b, which are both found to decrease with increasing temperature, and can be described in the following way: $a \left(T\right) = {a}^{25 ° C} {\left(p+\frac{q}{T - {T}_{ref}}\right)}^{u}$ $b \left(T\right) = {b}^{25 ° C} + {\left(r+\frac{s}{T - {T}_{ref}}\right)}^{v}$

It was found that the optimal *T_{ref}* for a and b are not very different, and a very similar agreement is obtained for a broad range of *T_{ref}.* Therefore, a determination of a(T) and b(T) can be based on the same *T_{ref}.*

It can be shown that - by applying the above formulae - viscosities of polyols based on the propoxylated starters for which a few experimental data are already available can be computed with mean deviations of less than 20%.

Among polyols as the main constituent of the A-component of PU foams, polyester polyols (PESOL) obtained via polycondensation of diols and dicarboxylic acids represent an important class. A crucial physical property of these PESOLs is their viscosity, which spans a huge range of values from below 30 mPa·s up to 30000 mPa·s. In most applications a viscosity above a predetermined threshold is not desired. In the following an equation for determining viscosities of polyester polyols as function of the molar mass or equivalently, OH number, temperature, functionality as well as used dicarboxylic acids and diols is provided that can be utilized, for example, as part of a material property model. Moreover, both equations are based on physical or empirically developed relation comprising one or more parameters that are trainable in a fitting procedure that fits the relations to respective training data.

As molar mass dependence, the functional form $η \left(M\right) = M exp \left(b⋅{M}^{α}+C\right)$ has been found to accurately reproduce the molar mass dependence of the viscosity of polyester polyols when choosing an exponent *α* > 0. The equation can be generalized to incorporate the temperature dependence of the viscosity by replacing parameters b and C by a temperature dependent version *A*+*B*/(*T-T*offset) and *C*+*D*/(*T-T*offset) $η \left(M, T\right) = M exp \left(\left(A+B/\left(T-Toffset\right)\right)⋅Mα+\left(C+D/\left(T-Toffset\right)\right)\right)$

. The five parameters *A,* B, C, D, Toffset can be trained based on respective experimental data. When changing diols and dicarboxylic acids used for the polyester polyol, the polyol's viscosity and its functionality changes. Varying diol structures can be integrated into the above formula by a diol dependent parameter *b*_{diol}. Acid dependency of the viscosity can be accounted for by parameters *a*_{acid} and *c*_{acid}. *A,* B, C, and D are independent of diol and acid structure. A functionality different from f= 2 can also be incorporated in the exponential leading to: $η \left(M, T\right) = M exp \left({b}_{diol}⋅{a}_{acid}\left(A+B/\left(T-Toffset\right)\right)⋅{\left(fM\right)}^{∧}α+{c}_{acid}⋅\left(C+D/\left(T-Toffset\right)\right)\right) .$

Please note that all three structure-dependent parameters either depend on the acid or the diol, but never on a combination "acid-diol". Consequently, the number of fit parameters only increases linearly when increasing the number of diols and acids.

For mixing polyols with different viscosity it has been found that the resulting viscosity can be determined for polyols with a viscosity that does not differ by more than a factor of 100 by: $f \left({η}_{mix}\right) = {\sum }_{i} {w}_{i} ln \left({η}_{i}\right)$

Here, *wᵢ* can be a mass fraction of the respective polyol in the mixture. For mixtures of constituents that differ in viscosity by e.g. a factor of 100 or even more, the following relation has been found to be accurate: ${η}_{mix}^{n} = {\sum }_{i} {y}_{i} {η}_{i}^{n} .$

Here, n is a fitting exponent.

In the following an example of a foaming process model is described. The foaming process model can be configured to model the rise and gelling of rigid foams based on their recipe and on the processing parameters under which the foams are manufactured. Besides the time dependent profiles of temperature, end group conversion and foam density also foaming characteristics can be modeled such as a gel time, a foam rise time, a demolding time, and a demolding cell gas pressure.

The gelling and rising of a polyurethane rigid foam can be modelled based on the following principles. A reaction kinetics of PU rigid foams can be described by an end group model which can distinguish the following group types of the utilized polyol Primary OH groups, Secondary OH groups, NCO groups in the position of the aromatic ring, NCO groups in the 4 position of the aromatic ring, amine groups. The following reactions can be considered reaction of all OH group types with all NCO group types to form urethane groups as gelling reaction, reaction of water with all NCO group types to form an amine and carbon dioxide as blowing reaction, reaction of all NCO groups with amine groups to form urea, reaction of urethane groups with all isocyanate groups to form allophanate groups. It can be assumed for all types of reactions that the reaction rate is first order in all reactants. For catalyzed systems it can be assumed that the reaction rate is proportional to the catalyst concentration for each single catalyst. The catalytic activity of the different catalysts is measured for the different reactions. For the non-catalyzed blowing and gelling reactions of the different reactive groups different preexponential factors and activation energies can be given. However, it can be assumed that preexponential factors and activation energies of all gelling reactions to not depend on whether an NCO-group is in the 2- or 4-position of the aromatic ring. The same assumption can be made for all blowing reactions. For the catalyzed gelling, it can be assumed that the reactivity neither depends on whether an OH-group is a primary or secondary one nor depends on whether an NCO group is in the 2- or the 4-position of the aromatic ring. For the catalyzed blowing reaction, it can be assumed that the reactivity does not depend on whether an NCO group is in the 2- or the 4-position of the aromatic ring. Hence, for each catalysts the catalytic activity for the blowing and gelling reaction can be described by one pair of preexponential factor and activation energy, respectively.

For the calculation of the evaporation of the reactive and physical blowing agents out of the polymer matrix the vapor-liquid equilibria of the different blowing agents at the polymer-cell gas interphase can be computed. Therefore, the vapor pressures of the pure blowing agents can be determined as a function of temperature. The vapor pressure of the blowing agents at the polymer-gas interface can be computed based on the pure component vapor pressure, the mass fraction of blowing agent in the polymer and its activity coefficient in the mixture. Activity coefficients can be assumed to be one. Besides the gas-liquid equilibrium data the diffusion coefficients of the blowing agents can be utilized together with a mass transfer model between the polymer matrix and the gas bubbles in the foam. It can be assumed that diffusion coefficients have an Arrhenius type temperature difference and are inversely proportional to the viscosity of the reaction mixture. For mass transfer between the polymer matrix and the gas bubbles a single but varying bubble size is assumed for all bubbles in the foam and effective mass transfer coefficient can be calculated.

The polymer reaction kinetics and hence the blowing agent evaporation can be influenced by the processing conditions of the foam that are defined by the processing parameters. The following processing aspects can be considered in the model: heat transfer from the foam to the surrounding mold via an effective heat transfer area defined by an effective heat transfer coefficient and a predefined mold temperature, delayed start of polymerization reactions due imperfect mixing of the polyol and isocyanate components via a reaction induction time, rise of foam under free rise conditions or under rise conditions in a closed mold, and definition of foam rise start conditions by assigning the temperatures and pressures of the feed streams that enter the reaction mixture.

Due to the gelling and blowing reactions a polymer network is formed. The microstructure of the polymer network is defined by the functionality of the employed polyols and isocyanates, by the stoichiometry of the system and by the amount of reacted end groups. Concentration of crosslinks, dangling and linear chains can be calculated as a function of time based on a conversion of the different end groups. It is noted that the method is suited for end groups with different reactivity and it can be applied to polyols and isocyanate of arbitrary functionality and composition of end groups. It can also consider the effect of allophanate, biuret and isocyanurate groups on the crosslinking concentration.

An increase of the molecular weight that is formed and later on the formation of crosslinks lead to a transition of the polymer from a viscous liquid to a solid material. The increase of the elastic modulus of the polymer goes along with its solidification. The elastic modulus of the polymer can be determined as a function of the crosslink concentration of the polymer and the process temperature.

Under closed mold rise conditions the foam gradually fills the mold. Once it has fully filled the mold the foam cannot expand anymore and pressure increase in the foam cells takes place if blowing agent is still evaporating. When the mold is opened, the foam can post expand due to the cell gas pressure. If the cell gas pressure is high and the modulus of the polymer is still low because of high temperature and/or low crosslink concentration the post expansion is significant and can lead to shape deviation of the demolded part. If bonds form under expanded conditions and the system cools down after the foaming process a residual shape deviation may occur because bonds have formed under stretched network conditions such that the part cannot relax entirely back to its state once the foam has cooled down and the cell pressure has redecreased again.

In the following an exemplary foaming process model is described that is configured for determining a crosslinking density of the PU foam for determining respective foaming characteristics. Rigid polyurethane (PU) foams are made up of highly crosslinked polyurethane bulk polymers. During processing of a polyurethane rigid foam part, the hydroxyl (OH-) groups of the polyol molecules and the isocyanate (NCO-) groups of the isocyanate molecules react with each other and form urethane bonds. Since the polyols and partly also the isocyanate molecules have a functionality higher than two, a network is gradually formed which leads to a transition of the reaction mixture from a viscous liquid to a solid material. Since the expansion of a foam part by evaporation of CO₂ or physical blowing agents is competing with the solidification (gelling) of the reaction mixture, knowledge with respect to the relation between the amount of reacted end groups and the microstructural parameters of the polymer network can be utilized for determining processing parameters like the crosslinking density. Based on the knowledge of the relation between microstructural parameters such as crosslink concentration or crosslink point concentration and mechanical properties of the bulk material, the foaming characteristics can be determined such as gel and demoulding time.

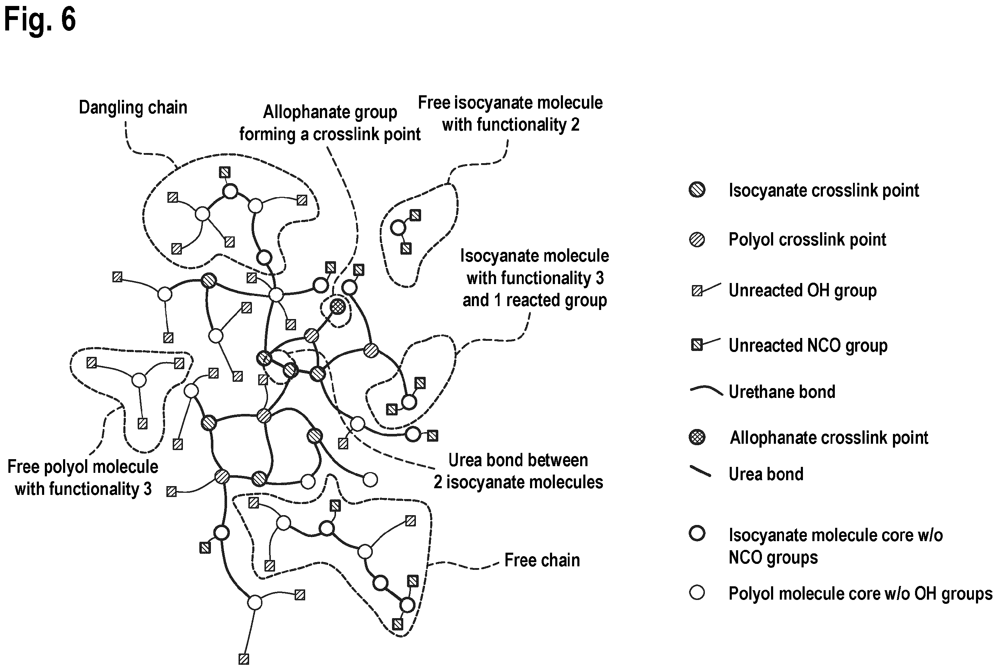

Fig. 6 shows a schematically and exemplarily sketch of a polymer network made up of polyol and isocyanate molecules of different functionalities. The drawing shows the (intrinsic) crosslink points of isocyanate molecules, and the (intrinsic) crosslink points of polyol molecules. Further, unreacted NCO and OH groups of the isocyanate and polyol molecules, respectively, are depicted. Also allophanate groups are depicted, a urethane bond between a polyol crosslink point and an isocyanate crosslink point are indicated. Note that two isocyanate crosslink point can directly be connected by a urea bond. Polyol and isocyanate molecules that are no crosslink points are also depicted.

In the following an example of an application property model is described. The application property model can be configured to determine rigid foam mechanical and thermal application properties. Preferably, isotropic and anisotropic foams can be considered. The model furthermore can be configured to determine the vapor pressure of each cell gas component and to check whether the partial pressure of the respective components is lower than its vapor pressure. If this is not the case its partial pressure and the total pressure of the cell gas mixture can be reduced. For example the thermal conductivity of the cell gas can be determined by a mole weighted algebraic averaging of the pure component thermal conductivities. The application property model can be configured to determine the Young's modulus and the compressive strength for isotropic and anisotropic rigid foams based on a storage modulus. The storage modulus can be determined based on empirically fitted equations and parameters that correlate the storage modulus with, for instance, a Rubbery modulus, the glass transition temperature and a glass transition range. The Young's modulus can then be determined based on the storage modulus and the Poisson ration.

Further the application property model can also be configured to determine the change of a thermal conductivity of a PU foam and the change of its thickness with time. The determination can be based on a one dimensional model which assumes a foam sample with an infinite extension perpendicular to thickness direction of a foam sample. It furthermore can be assumed in the model, that there is no diffusion barrier on top of the two sides of the sample and that the foam sample itself is homogeneous. The diffusion of the different cell gas components in the foam can be determined. From that the average cell gas composition and pressure as a function of time can be determined which can be the basis for the determination of change of the thermal conductivity and the thickness of the foam sample. The time dependent average cell gas composition can be used together with the thermal application property model to determine a change of thermal conductivity of the PU foam. The cell gas pressure change leads to a change of the difference pressure between the cell gas and the ambient atmosphere. Dependent on the stiffness of the foam sample this leads to a more or less pronounced expansion or shrinkage of the sample. The mechanical application property model can be configured to calculate from the pressure change the resulting sample deformation.

Other variations to the disclosed embodiments can be understood and effected by those skilled in the art in practicing the claimed invention, from a study of the drawings, the disclosure, and the appended claims.

For the processes and methods disclosed herein, the operations performed in the processes and methods may be implemented in differing order. Furthermore, the outlined operations are only provided as examples, and some of the operations may be optional, combined into fewer steps and operations, supplemented with further operations, or expanded into additional operations without detracting from the essence of the disclosed embodiments.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the receiving of a digital representation, determining of material properties, validation a start recipe, etc. performed by one or several units or devices can be performed by any other number of units or devices. These procedures can be implemented as program code means of a computer program and/or as dedicated hardware.

A computer program product may be stored/distributed on a suitable medium, such as an optical storage medium or a solid-state medium, supplied together with or as part of other hardware, but may also be distributed in other forms, such as via the Internet or other wired or wireless telecommunication systems.

Any units described herein may be processing units that are part of a classical computing system. Processing units may include a general-purpose processor and may also include a field programmable gate array (FPGA), an application specific integrated circuit (ASIC), or any other specialized circuit. Any memory may be a physical system memory, which may be volatile, non-volatile, or some combination of the two. The term "memory" may include any computer-readable storage media such as a non-volatile mass storage. If the computing system is distributed, the processing and/or memory capability may be distributed as well. The computing system may include multiple structures as "executable components". The term "executable component" is a structure well understood in the field of computing as being a structure that can be software, hardware, or a combination thereof. For instance, when implemented in software, one of ordinary skill in the art would understand that the structure of an executable component may include software objects, routines, methods, and so forth, that may be executed on the computing system. This may include both an executable component in the heap of a computing system, or on computer-readable storage media. The structure of the executable component may exist on a computer-readable medium such that, when interpreted by one or more processors of a computing system, e.g., by a processor thread, the computing system is caused to perform a function. Such structure may be computer readable directly by the processors, for instance, as is the case if the executable component were binary, or it may be structured to be interpretable and/or compiled, for instance, whether in a single stage or in multiple stages, so as to generate such binary that is directly interpretable by the processors. In other instances, structures may be hard coded or hard wired logic gates, that are implemented exclusively or near-exclusively in hardware, such as within a field programmable gate array (FPGA), an application specific integrated circuit (ASIC), or any other specialized circuit. Accordingly, the term "executable component" is a term for a structure that is well understood by those of ordinary skill in the art of computing, whether implemented in software, hardware, or a combination. Any embodiments herein are described with reference to acts that are performed by one or more processing units of the computing system. If such acts are implemented in software, one or more processors direct the operation of the computing system in response to having executed computer-executable instructions that constitute an executable component. Computing system may also contain communication channels that allow the computing system to communicate with other computing systems over, for example, network. A "network" is defined as one or more data links that enable the transport of electronic data between computing systems and/or modules and/or other electronic devices. When information is transferred or provided over a network or another communications connection, for example, either hardwired, wireless, or a combination of hardwired or wireless, to a computing system, the computing system properly views the connection as a transmission medium. Transmission media can include a network and/or data links which can be used to carry desired program code means in the form of computer-executable instructions or data structures and which can be accessed by a general-purpose or special-purpose computing system or combinations. While not all computing systems require a user interface, in some embodiments, the computing system includes a user interface system for use in interfacing with a user. User interfaces act as input or output mechanism to users for instance via displays.

Those skilled in the art will appreciate that at least parts of the invention may be practiced in network computing environments with many types of computing system configurations, including, personal computers, desktop computers, laptop computers, message processors, hand-held devices, multi-processor systems, microprocessor-based or programmable consumer electronics, network PCs, minicomputers, main-frame computers, mobile telephones, PDAs, pagers, routers, switches, datacenters, wearables, such as glasses, and the like. The invention may also be practiced in distributed system environments where local and remote computing system, which are linked, for example, either by hardwired data links, wireless data links, or by a combination of hardwired and wireless data links, through a network, both perform tasks. In a distributed system environment, program modules may be located in both local and remote memory storage devices.

Those skilled in the art will also appreciate that at least parts of the invention may be practiced in a cloud computing environment. Cloud computing environments may be distributed, although this is not required. When distributed, cloud computing environments may be distributed internationally within an organization and/or have components possessed across multiple organizations. In this description and the following claims, "cloud computing" is defined as a model for enabling on-demand network access to a shared pool of configurable computing resources, e.g., networks, servers, storage, applications, and services. The definition of "cloud computing" is not limited to any of the other numerous advantages that can be obtained from such a model when deployed. The computing systems of the figures include various components or functional blocks that may implement the various embodiments disclosed herein as explained. The various components or functional blocks may be implemented on a local computing system or may be implemented on a distributed computing system that includes elements resident in the cloud or that implement aspects of cloud computing. The various components or functional blocks may be implemented as software, hardware, or a combination of software and hardware. The computing systems shown in the figures may include more or less than the components illustrated in the figures and some of the components may be combined as circumstances warrant.

Any reference signs in the claims should not be construed as limiting the scope.

The invention refers to a method for assisting in generating a recipe for a rigid polyurethane foam. A start recipe is associated with one or more ingredients, wherein the ingredients comprise ingredients of a polyol-component, and ingredients of an isocyanate-component comprising an isocyanate. Material properties of the polyol and/or the polyol-component are determined based on the start recipe. The start recipe is validated based on the determined material properties of the polyol and/or the polyol-component. If the validation result is positive, process parameters associated with the parameters utilized in a foaming process are received. Foam characteristics are determined based on the validated start recipe and the process parameters. Application properties of the rigid polyurethane foam are determined based on the determined foam characteristics. The application properties are provided for assisting in generating a final recipe for a rigid polyurethane foam comprising target application properties.

## Claims

1. A method, in particular, a computer implemented method, for assisting in generating a recipe for a rigid polyurethane foam comprising one or more target application properties, wherein the method comprises:
receiving a digital representation of a start recipe for producing a rigid polyurethane foam, wherein the start recipe is associated with one or more ingredients utilizable for producing the rigid polyurethane foam, wherein the ingredients comprise ingredients of a polyol-component comprising a polyol and a blowing agent, and ingredients of an isocyanate-component comprising an isocyanate,
determining one or more material properties of the polyol and/or the polyol-component based on the start recipe,
validating the start recipe based on the determined one or more material properties of the polyol and/or the polyol-component,
if the validation result is positive, receive one or more process parameters associated with the parameters utilized in a foaming process of the polyurethane foam,
determine one or more foam characteristics based on the validated start recipe and the one or more process parameters, wherein the one or more foam characteristics are associated with structural and compositional characteristics of the rigid polyurethane foam,
determine one or more application properties of the rigid polyurethane foam based on the determined one or more foam characteristics, and
providing the one or more application properties for assisting in generating a final recipe for a rigid polyurethane foam comprising one or more target application properties.

2. The method according to claim 1, wherein the determining of the one or more material properties comprises determining a) a chemical composition of the polyol in the polyol component based on the start recipe and b) determining the one or more material properties based on the chemical composition of the polyol.

3. The method according to claim 2, wherein determining the chemical composition comprises determining the types of monomers of the polyol and the amount of the respective types of monomers in the polyol.

4. The method according to claim 1, wherein the application properties comprise mechanical properties and/or thermal properties of the polyurethane foam.

5. The method according to any of the preceding claims, wherein the method comprises, if the validation of the polyol and/or the polyol-component is negative, generating a new start recipe comprising a different polyol-component and performing the steps of the method based on the new start recipe.

6. The method according to claim 5, wherein the method further comprises receiving one or more recipe constraints indicative of one or more constraints with respect to the polyol and/or the polyol-component, wherein the new start recipe is generated based on the received recipe constraints.

7. The method according to any of the preceding claims, wherein the method further comprises determining one or more insulation properties of the polyurethane foam based on the validated start recipe and the one or more foam characteristics.

8. The method according to any of the preceding claims, wherein the one or more material properties comprise at least one of a blowing agent solubility for one or more predetermined blowing agents, a viscosity, an isocyanate compatibility with one or more predetermined isocyanates, and a glass transition temperature of an unfoamed bulk polymer in the polyurethane foam.

9. The method according to any of the preceding claims, wherein the one or more foam characteristics comprise at least one of a foam density, a degree of cross-linking, a cell gas composition, an average foam cell diameter, a cell shape, and a cell gas pressure.

10. The method according to any of the preceding claims, wherein the method further comprises determining one or more foaming characteristics, preferably, comprising at least one of a foam rise time, gelling time, curing speed, a foaming speed, and demolding time, based on the validated start recipe and the one or more process parameters.

11. The method according to any of the preceding claims, wherein the method comprises receiving one or more target constraints with respect to the one or more application properties, wherein the one or more target constraints are to be fulfilled by the polyurethane foam, wherein the method further comprises further validating the validated start recipe with respect to the target constraints based on the determined one or more application properties and providing a final recipe based on the validation.

12. The method according to claim 11, wherein, the method comprises a) if the validation is negative, generating a new start recipe and performing the steps of the method based on the new start recipe, or b) if the validation is positive, providing the validated start recipe as final recipe for controlling and/or monitoring a production of the polyurethane foam.

13. The method according to any of claims 11 and 12, wherein the new start recipe comprising at least one of a polyol-component, catalyst, amount of one or more components and/or process parameter different from the start recipe.

14. An apparatus for assisting in generating a recipe for a rigid polyurethane foam comprising one or more target application properties, wherein the apparatus comprises one or more processors configured to perform a method comprising:
receiving a digital representation of a start recipe for producing a rigid polyurethane foam, wherein the start recipe is associated with one or more ingredients utilizable for producing the polyurethane foam, wherein the ingredients comprise ingredients of a polyol-component comprising at least a polyol, and a blowing agent, and ingredients of an isocyanate-component comprising an isocyanate,
determining one or more material properties of the polyol and/or the polyol-component based on the start recipe,
validating the start recipe based on the determined one or more material properties of the polyol and/or the polyol-component,
if the validation result is positive, receiving one or more process parameters associated with the parameters utilized in a foaming process of the polyurethane foam, determining one or more foam characteristics based on the validated start recipe and the one or more process parameters, wherein the one or more foam characteristics are associated with structural and compositional characteristics of the polyurethane foam,
determining one or more application properties of the polyurethane foam based on the determined foam characteristics, and
providing the one or more application properties for assisting in generating a final recipe for a polyurethane foam comprising one or more target application properties.

15. A computer program product configured for causing an apparatus according to claim 14 to perform a method according to any of claims 1 to 13 when the computer program product is carried out by the apparatus.
